(19) Europäisches Patentamt
European Patent Office
Office européen des brevets

(11) Publication number: **0 125 695**
**B1**

(12) # EUROPEAN PATENT SPECIFICATION

(45) Date of publication of patent specification: **22.07.87**

(51) Int. Cl.⁴: **C 07 C 49/697**, A 61 K 31/12

(21) Application number: **84105550.2**

(22) Date of filing: **16.05.84**

(54) **2-Bromo-1-tetralone derivatives.**

(30) Priority: **16.05.83 JP 85433/83**

(43) Date of publication of application:
**21.11.84 Bulletin 84/47**

(45) Publication of the grant of the patent:
**22.07.87 Bulletin 87/30**

(84) Designated Contracting States:
**DE FR GB**

(56) References cited:
**CH-A- 520 645**
**DE-A-2 640 358**
**US-A-3 919 316**

(73) Proprietor: **KIRIN BEER KABUSHIKI KAISHA**
**26-1 Jingumae 6-chome**
***Shibuya-ku Tokyo-To (JP)***

(72) Inventor: **Sato, Yuko Kirin Beer K.K.**
**Kaihatsu Kagaku Kenkyusho 3, Miyahara-Cho**
**Takasaki-Shi Gunma-Ken (JP)**
Inventor: **Mizobuchi, Shigeyuki Kirin Beer K.K.**
**Kaihatsu Kagaku Kenkyusho 3, Miyahara-Cho**
**Takasaki-Shi Gunma-Ken (JP)**
Inventor: **Tanabe, Kozo Kirin Beer K.K.**
**Kaihatsu Kagaku Kenkyusho 3, Miyahara-Cho**
**Takasaki-Shi Gunma-Ken (JP)**
Inventor: **Inoue, Hideo Kirin Beer K.K.**
**Kaihatsu Kagaku Kenkyusho 3, Miyahara-Cho**
**Takasaki-Shi Gunma-Ken (JP)**

(74) Representative: **Reichel, Wolfgang, Dipl.-Ing. et al**
**Reichel und Reichel Parkstrasse 13**
**D-6000 Frankfurt am Main 1 (DE)**

**EP 0 125 695 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

BACKGROUND OF THE INVENTION

The present invention relates to novel 2-bromo-1-tetralone derivatives.

Generally, the physiological activities of chemicals depend greatly on their chemical structures. There has been a constant demand, therefore, for chemical substances having structures which differ from those of known compounds.

SUMMARY OF THE INVENTION

This invention presents one contribution toward meeting the above mentioned demand.

More particularly, this invention provides 2-bromo-1-tetralone derivatives of the formula (I):

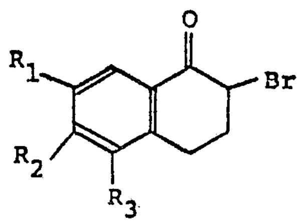

(I)

wherein $R_1$, $R_2$ and $R_3$ form any one of the following combinations (A), (B) and (C):

(A) $R_1 = R_2 = Cl$ and $R_3 = H$;

(B) $R_1 = R_3 = Cl$ and $R_2 = H$; and

(C) $R_1 = F$ and $R_2 = R_3 = H$.

BRIEF DESCRIPTION OF THE DRAWINGS

In the drawings:

FIG. 1 is a diagram showing the infrared absorption spectrum of 2-bromo-6,7-dichloro-1-tetralone (in chloroform);

FIG. 2 is a diagram showing the infrared absorption spectrum of 2-bromo-5,7-dichloro-1-tetralone (in chloroform);

FIG. 3 is a diagram showing the infrared absorption spectrum of 2-bromo-7-fluoro-1-tetralone (in chloroform);

FIG. 4 is a diagram showing the visible/ultraviolet absorption spectrum of 2-bromo-6,7-dichloro-1-tetralone (in methanol);

FIG. 5 is a diagram showing the visible/ultraviolet absorption spectrum of 2-bromo-5,7-dichloro-1-tetralone (in methanol); and

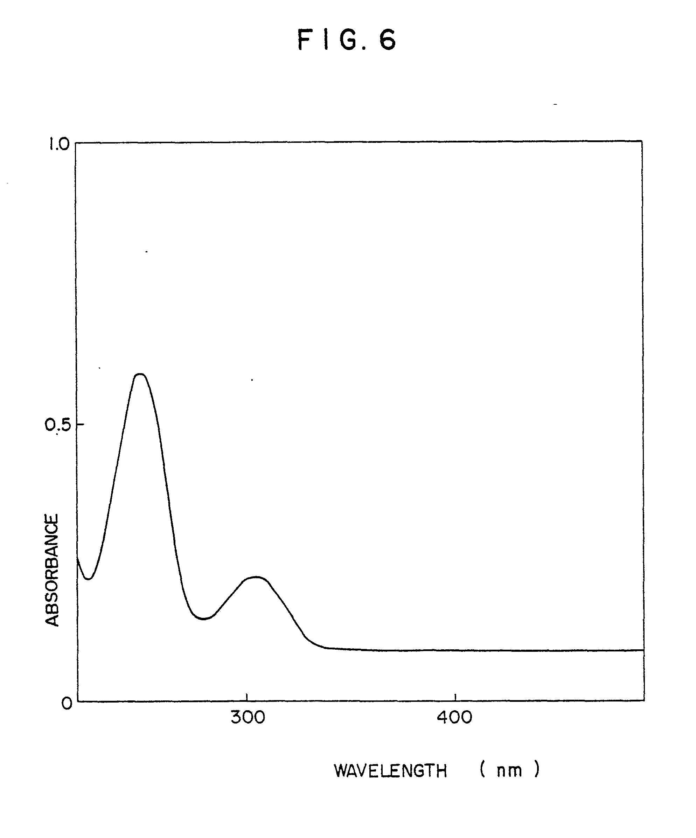

FIG. 6 is a diagram showing the visible/ultraviolet absorption spectrum of 2-bromo-7-fluoro-1-tetralone (in methanol).

DETAILED DESCRIPTION OF THE INVENTION

2-Bromo-1-tetralone derivatives

The 2-bromo-1-tetralone derivatives according to the present invention have a chemical structure as shown by the above formula (I) wherein the substituents $R_1$, $R_2$ and $R_3$ form any one of the three combinations (A), (B) and (C), forming respectively 2-bromo-6,7-dichloro-1-tetralone, 2-bromo-5,7-dichloro-1-tetralone and 2-bromo-7-fluoro-1-tetralone.

The term "2-bromo-1-tetralone derivative(s)" used herein refers to any one of these compounds or a mixture of two or more thereof.

The physicochemical properties of these 2-bromo-1-tetralone derivatives are summarized in Table 1.

TABLE 1
Physicochemical Properties of 2-Bromo-1-Tetralone Derivatives

| | 2-bromo-6,7-dichloro-1-tetralone | 2-bromo-5,7-dichloro-1-tetralone | 2-bromo-7-fluoro-1-tetralone |
|---|---|---|---|
| Elementary analysis (%) | (Found) C = 40.91, H = 2.14<br>(Calcd.) C = 40.86, H = 2.40 | (Found) C = 40.89, H = 2.25<br>(Calcd.) C = 40.86, H = 2.40 | (Found) C = 49.44, H = 3.18<br>(Calcd.) C = 49.41, H = 3.32 |
| Mass spectrum (M/e) (abundant peak only) | 294 ($M^+$)<br>214 ($M^+ - 80$) | 294 ($M^+$)<br>214 ($M^+ - 80$) | 242 ($M^+$)<br>162 ($M^+ - 80$) |
| Infrared absorption spectrum (in chloroform) $(-\overset{O}{\overset{\|}{C}}-)$ | FIG. 1<br>(1690 $cm^{-1}$) | FIG. 2<br>(1695 $cm^{-1}$) | FIG. 3<br>(1690 $cm^{-1}$) |
| Visible/ultraviolet absorption spectrum $\lambda_{max}$ (nm) [$\varepsilon_{max}$] | FIG. 4<br>261 [13,800]<br>306 [2,530] | FIG. 5<br>256 [8,070]<br>311 [1,800] | FIG. 6<br>251 [10,300]<br>304 [2,720] |
| Appearance | Pale yellowish orange<br>Plate crystal | Colorless<br>Needle crystal | Yellow<br>Oily |
| Melting point (°C) | 88.0—89.5 | 106—109 | — |

TABLE 1 (continued)

| | 2-bromo-6,7-dichloro-1-tetralone | 2-bromo-5,7-dichloro-1-tetralone | 2-bromo-7-fluoro-1-tetralone |
|---|---|---|---|
| NMR spectrum (100 MHz, in deuterochloroform) (δ ppm)<br>$C_2 - H$<br>$C_8 - H$ | 4.70 (t., 1H, J = 4.0)<br>8.15 (s., 1H) | 4.70 (t., 1H, J = 4.0)<br>7.98 (d., 1H, J = 2.2) | 4.67 (t., 1H, J = 4.1)<br>7.81 (d., 1H, J = 1.0) |
| Thin layer chromatography<br>Rf* Chloroform: Hexane = 3:1 | 0.55 | 0.60 | 0.56 |
| Solubility in solvent | Easily soluble in methanol, ethanol, acetone, chloroform and ether; sparingly soluble in n-hexane; and insoluble in water (all of the derivatives) | | |
| Structural formula | 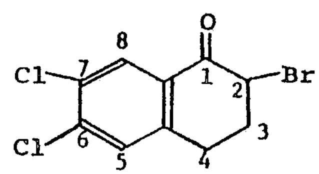 | 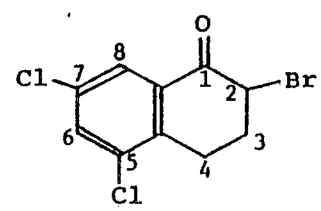 | 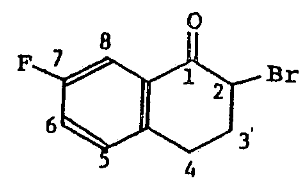 |

* Silica gel plate (ART 5721) supplied by Merck & Co., Inc.) was used.

Production of 2-bromo-1-tetralone derivatives

2-Bromo-1-tetralone derivatives can be produced by any process suitable for the desired purpose with respect to the introduction of substituents or the formulation of chemical structures. A specific example of such processes is illustrated below.

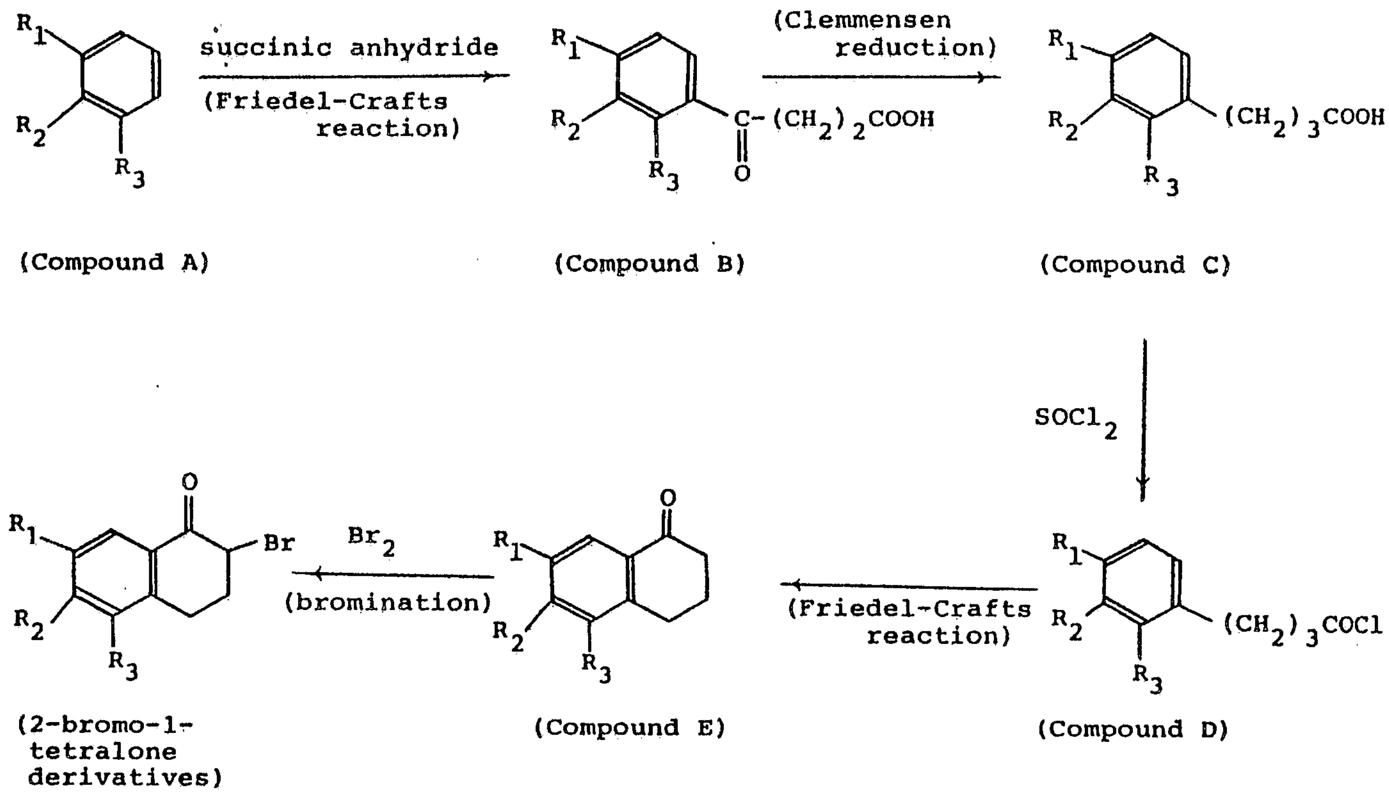


Note: $R_1$, $R_2$ and $R_3$ are the same as defined in the formula (I) shown hereinbefore.

First, Compound A and succinic anhydride are subjected to Friedel-Crafts reaction to obtain Compound B. The Compound B thus obtained is caused to undergo Clemmensen reduction to obtain Compound C, which is reacted with thionyl chloride to produce Compound D. The Compound D is then cyclized by Friedel-Crafts reaction to produce Compound E. This Compound E is reacted with equimolar bromine in the presence of ethyl ether or carbon disulfide to obtain 2-bromo-1-tetralone derivatives.

Utility of 2-bromo-1-tetralone derivatives

The 2-bromo-1-tetralone derivatives in accordance with the present invention have antitumor activity coupled with antifungal activity, and are therefore useful as a medicine.

Physiological activities

(1) Antitumor activity

2-Bromo-1-tetralone derivatives exhibit antitumor activity against leukemia of subject animals. For example, into $CDF_1$ mice were intraperitoneally transplanted P388 leukemia $1 \times 10^6$ cells/mouse as a suspension, and 2-bromo-6,7-dichloro-1-tetralone which is typical of the compounds of the present invention was administered to the mice 1 day and 5 days respectively after the transplantation. The effect of the test compound was evaluated in terms of the increase of life span (%) as calculated by determining the survival days of the control group consisting of mice administered with physiological saline solution as 100. The results obtained were as shown in Table 2 below.

TABLE 2
Antitumor Activity of 2-Bromo-6,7-Dichloro-1-Tetralone

| Dose (mg/kg/day) | Increase of life span (%) |
|---|---|
| 10.0 | 129 |
| 50.0 | 140 |

(2) Antifungal activity

The 2-bromo-1-tetralone derivatives according to this invention exhibit antimicrobial activity against various fungi, and the minimum inhibitory concentration (MIC) of these compounds obtained by the Yamano et al. method (Yamano and Henmi, Shinkin-Shi (Journal of Fungi), *10*, 68(1969)) was as shown in Table 3, in which data on griseofulvin as a control are also set forth.

TABLE 3
Minimum Inhibitory Concentration of 2-Bromo-1-Tetralone Derivatives

| Microorganism / Compound | Minimum Inhibitory Concentration (MIC) [μg/ml] | | |
|---|---|---|---|
| | Trichophyton mentagrophytes | Trichophyton asteroides | Candida albicans |
| 2-bromo-6,7-dichloro-1-tetralone | 0.78 | 0.78—1.56 | 1.56 |
| 2-bromo-5,7-dichloro-1-tetralone | 1.56 | 1.56 | 1.56 |
| 2-bromo-7-fluoro-1-tetralone | 3.13 | 3.13 | 6.25 |
| griseofulvin | 6.25 | 6.25 | >200 |

The minimum inhibitory concentration was determined as follows.

Method of Determination of MIC

(a) Culture medium

Sabouraud's agar medium was used as a culture medium. (A liquid culture medium was prepared from the same ingredients except for agar).

| | |
|---|---|
| Glucose | 40 g |
| Peptone | 10 g |
| Agar | 15 g |
| Distilled water | 1 liter |

This culture medium was prepared by dissolving the above ingredients under heat, and the medium thus prepared was sterilized under the conditions of 1.05 bar (15 lbs./in$^2$) for 15 minutes.

(b) Preparation of suspension containing microorganism to be tested

The microorganisms to be tested were fungi of the species *Trichophyton mentagrophytes* (A), *Trichophyton asteroides* (B) and *Candida albicans* (C). Each of these microorganisms was cultivated on the corresponding slant medium at 27°C for 7 days. A loopful of each of the microorganisms (A), (B) and (C) was triturated in a mortar, charged into a test tube containing 10 ml of physiological saline solution, and suspended therein with a thermomixer. Each of the suspensions thus prepared was used in the test.

(c) MIC on agar medium

9 ml of the culture medium and 1 ml of a dilute sample were thoroughly admixed with each other in a Petri dish to prepare an agar plate, which was then streaked with the suspension containing each of the microorganisms to be tested by using a sterile platinum wire loop. The microorganisms (A), (B) and (C) thus inoculated on the agar plates were cultivated at 27°C for 7 days, and thereafter the MIC thereof was determined.

The 2-bromo-1-tetralone derivatives according to the present invention exhibit antifungal activity superior to that of known antifungal agents such as griseofulvin. Examples of fungi whose growth can be inhibited by the 2-bromo-1-tetralone derivatives of this invention are Trichophyton microorganisms such as *Trichophyton mentagrophytes* and *Trichophyton asteroides,* and yeast such as *Candida albicans.* Among these, the compounds of the present invention are most effective against Trichophyton microorganisms.

(3) Toxicity

The toxicity of the compounds of the present invention as evaluated in terms of $LD_{50}$ of 2-bromo-6,7-dichloro-1-tetralone to mice is tabulated below.

| Route of Administration | $LD_{50}$ (mg/kg) |
|---|---|
| Oral | >1,500 |
| Intraperitoneal | 820 |
| Subcutaneous | >1,500 |

Applicability of 2-bromo-1-tetralone derivatives as antitumor agents

The 2-bromo-1-tetralone derivatives in accordance with the present invention can be used as antitumor agents or pharmaceutical compositions for treatment of tumors.

2-Bromo-1-tetralone derivatives as antitumor agents can be administered via any route suited for the desired purpose in a dosage form determined by the particular route of administration selected. Ordinarily, the compounds diluted with pharmaceutically acceptable carriers or diluents are administered as drugs.

A typical method of administering 2-bromo-1-tetralone derivatives as antitumor agents is by injection of the solution thereof in distilled water for injection use or in physiological saline solution. Examples of injection are intraperitoneal injection, subcutaneous injection, intravenous or intraarterial injection, and topical administration in the case of animals; and intravenous or intraarterial injection, and topical administration in the case of humans.

The doses of 2-bromo-1-tetralone derivatives are determined in accordance with the results of animal experiments and varying circumstances in such a manner that the total of the doses given continuously or intermittently in each case will not exceed a specific limit. Needless to say, particular doses required vary depending on the mode of administration; situations of patients or animals to be treated, such as age, body weight, sex and susceptibility; food; times of administration; concomitant drugs; and conditions of

patients or animals or severity of their diseases. The optimum doses and the frequency of administration under certain conditions must be determined by experts' optimum dose determination tests on the basis of the above-mentioned factors.

## Experimental Example

Production of 2-Bromo-6,7-Dichloro-1-Tetralone

(a) Preparation of β-(3,4-dichloro)benzoylpropionic acid

To 19.4 g of o-dichlorobenzene were added 10 g of succinic anhydride and then 29.3 g of powdery anhydrous aluminum chloride. The mixture was refluxed with stirring for approximately 1 hour, and thereafter water was added to decompose an aluminum complex salt. The resultant mixture was extracted with ethyl ether under acidic conditions, and a fraction soluble in an aqueous alkali solution was extracted from the ethyl ether extract. The aqueous solution thus obtained was acidified to form a precipitate. This precipitate was recrystallized from hot water, filtered off and dried to obtain 14.23 g of β-(3,4-dichloro)benzoylpropionic acid. The yield was 57.6%.

(b) Preparation of γ-(3,4-dichloro)phenylbutyric acid

To 7 g of the β-(3,4-dichloro)benzoylpropionic acid obtained in the step (a) were added 16.8 g of zinc amalgam, 11 ml of water, 30 ml of concentrated hydrochloric acid and 14 ml of toluene. The mixture was then refluxed. To the mixture was further added 7 ml of concentrated hydrochloric acid every 6 hours during the reflux until the reaction would not proceed further. After cooling, the reaction solution was extracted with ethyl ether, concentrated to dryness under reduced pressure and purified by silica gel column chromatography to obtain 4.82 g of γ-(3,4-dichloro)phenylbutyric acid. The yield was 73.0%.

(c) Preparation of 6,7-dichloro-1-tetralone

To 1.9 g of the γ-(3,4-dichloro)phenylbutyric acid obtained in the step (b) was added 6 ml of thionyl chloride, and the mixture was caused to react in solution state. Thereafter, the thionyl chloride was removed from the reaction solution to which 10 ml of carbon disulfide and 1.8 g of powdery anhydrous aluminum chloride were added. The resultant solution was refluxed with stirring for 10 minutes; an aluminum complex salt was decomposed with water; and the solution was then extracted with ethyl ether. The ethyl ether extract obtained was concentrated to dryness under reduced pressure and purified by silica gel column chromatography to produce 1.5 g of 6,7-dichloro-1-tetralone. The yield was 85.4%.

(d) Production of 2-bromo-6,7-dichloro-1-tetralone

1 g of the 6,7-dichloro-1-tetralone obtained in the step (c) was dissolved in 10 ml of ethyl ether or carbon disulfide, and 0.25 ml of bromine was added dropwise to the resulting solution at room temperature with stirring. After 30 minutes' reaction, water was added and the reaction solution was extracted with ethyl ether. The ethyl ether extract obtained was neutralized with an aqueous solution of sodium hydrogencarbonate, and then concentrated to dryness under reduced pressure to obtain 1.43 g of a crude product of 2-bromo-6,7-dichloro-1-tetralone. The yield was 99.6%.

This crude product was purified by silica gel column chromatography and subsequently recrystallized from n-hexane to produce 1.10 g of 2-bromo-6,7-dichloro-1-tetralone in the form of a pale yellowish orange plate crystal. The yield was 76.5%.

Elemental analysis

*Calcd.* C: 40.86% H: 2.40% (for $C_{10}H_7BrCl_2O$)

*Found* C: 40.91% H: 2.14%

**Claims**

1. 2-Bromo-1-tetralone derivatives of the formula

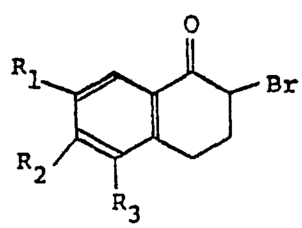

wherein $R_1$, $R_2$ and $R_3$ form any one of the following combinations (A), (B) and (C):

(A) $R_1 = R_2 = Cl$ and $R_3 = H$;

(B) $R_1 = R_3 = Cl$ and $R_2 = H$; and

(C) $R_1 = F$ and $R_2 = R_3 = H$.

2. Process for the preparation of said 2-Bromo-1-tetralone derivatives of claim 1, characterized by subjecting a compound of Formula A

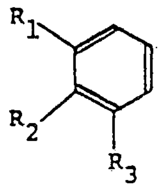

and succinic anhydride to Friedel-Crafts reaction to form a compound of Formula B

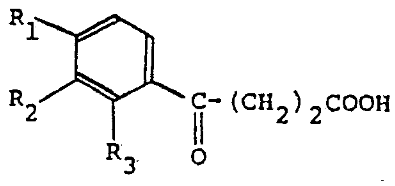

subjecting the compound of Formula B to Clemensen reduction to obtain a compound of Formula C

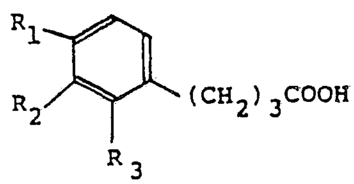

reacting the compound of Formula C with thionyl chloride to produce a compound of Formula D

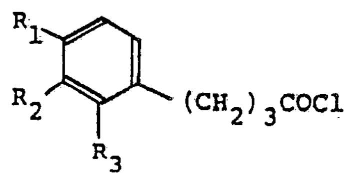

cyclizing the compound of Formula D by Friedel-Crafts reaction to obtain a compound of Formula E

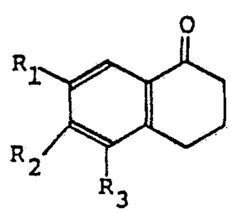

wherein in each case the substituents $R_1$, $R_2$, $R_3$ are as defined in claim 1, and brominating the compound of Formula E.

3. Pharmaceutical preparation containing a compound as claimed in claim 1 as an active ingredient together with usual pharmaceutical carriers and/or diluents.

**Patentansprüche**

1. 2-Brom-1-tetralon-Derivate der Formel

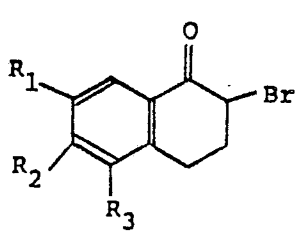

(I)

worin $R_1$, $R_2$ und $R_3$ eine Bedeutung gemäß einer der folgenden Kombinationen (A), (B) und (C) besitzen:

(A) $R_1 = R_2 = Cl$ und $R_3 = H$;

(B) $R_1 = R_3 = Cl$ und $R_2 = H$; und

(C) $R_1 = F$ und $R_2 = R_3 = H$.

2. Verfahren zur Herstellung der 2-Brom-1-tetralon-Derivate gemäß Anspruch 1, dadurch gekennzeichnet, daß man eine Verbindung der Formel A

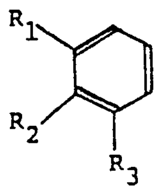

und Bernsteinsäureanhydrid unter Bildung einer Verbindung gemäß B

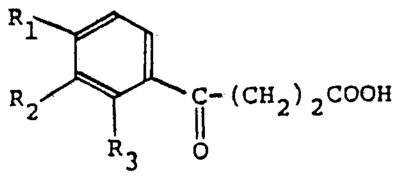

einer Friedel-Crafts Reaktion unterwirft, die Verbindung der Formel B unter Bildung einer Verbindung der Formel C

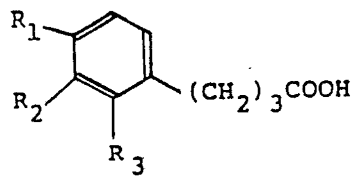

einer Clemmensen Reduktion unterwirft, die Verbindung der Formel C mit Thionylchlorid unter Bildung einer Verbindung der Formel D

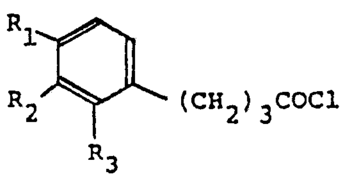

umsetzt, die Verbindung der Formel D mit Hilfe einer Friedel-Crafts Reaktion unter Bildung einer Verbindung der Formel E

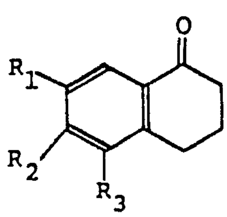

cyclisiert, wobei in jedem Falle die Substituenten $R_1$, $R_2$ und $R_3$ die in Anspruch 1 angegebene Bedeutung besitzen, und die Verbindung der Formel E bromiert.

3. Pharmazeutisches Präparat, enthaltend eine Verbindung gemäß Anspruch 1 als aktiven Bestandteil zusammen mit üblichen pharmazeutischen Trägern und bzw. oder Verdünnungsmitteln.

**Revendications**

1. Dérivés de 2-bromo-1-tétralone de la formule (I):

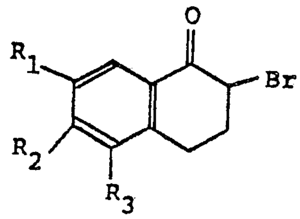

(I)

caractérisés en ce que $R_1$, $R_2$ et $R_3$ forment l'une quelconque des combinaisons suivantes (A), (B) et (C):
(A) $R_1 = R_2 = Cl$ et $R_3 = H$;
(B) $R_1 = R_3 = Cl$ et $R_2 = H$; et
(C) $R_1 = F$ et $R_2 = R_3 = H$.

2. Procédé de préparation des dérivés de 2-bromo-1-tétralone selon la revendication 1, caractérisé par le fait que l'on soumet un composé de formule A

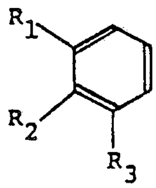

et l'anhydride succinique à une réaction de Friedel-Crafts pour former un composé de formule B

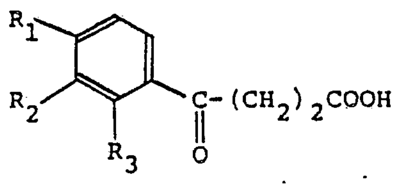

que l'on soumet le composé de formule B à la réduction de Clemmensen pour obtenir un composé de formule C

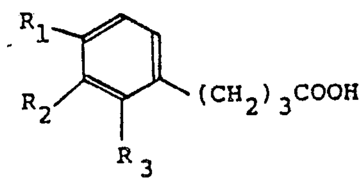

que l'on fait réagir le composé de formule C sur le chlorure de thionyle pour obtenir un composé de formule D

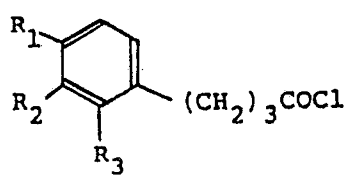

que l'on cyclise le composé de formule D par réaction de Friedel-Crafts pour obtenir un composé de formule E

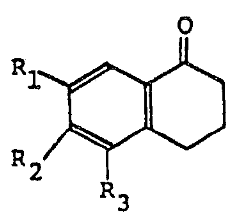

dans lesquelles, dans chaque cas, les substituants $R_1$, $R_2$, $R_3$ sont comme défini à la revendication 1, et que l'on brome le composé de formule E.

3. Préparation pharmaceutique contenant un composé selon la revendication 1 comme ingrédient actif, en même temps que des véhicules et/ou diluants pharmaceutiques usuels.

FIG. 1

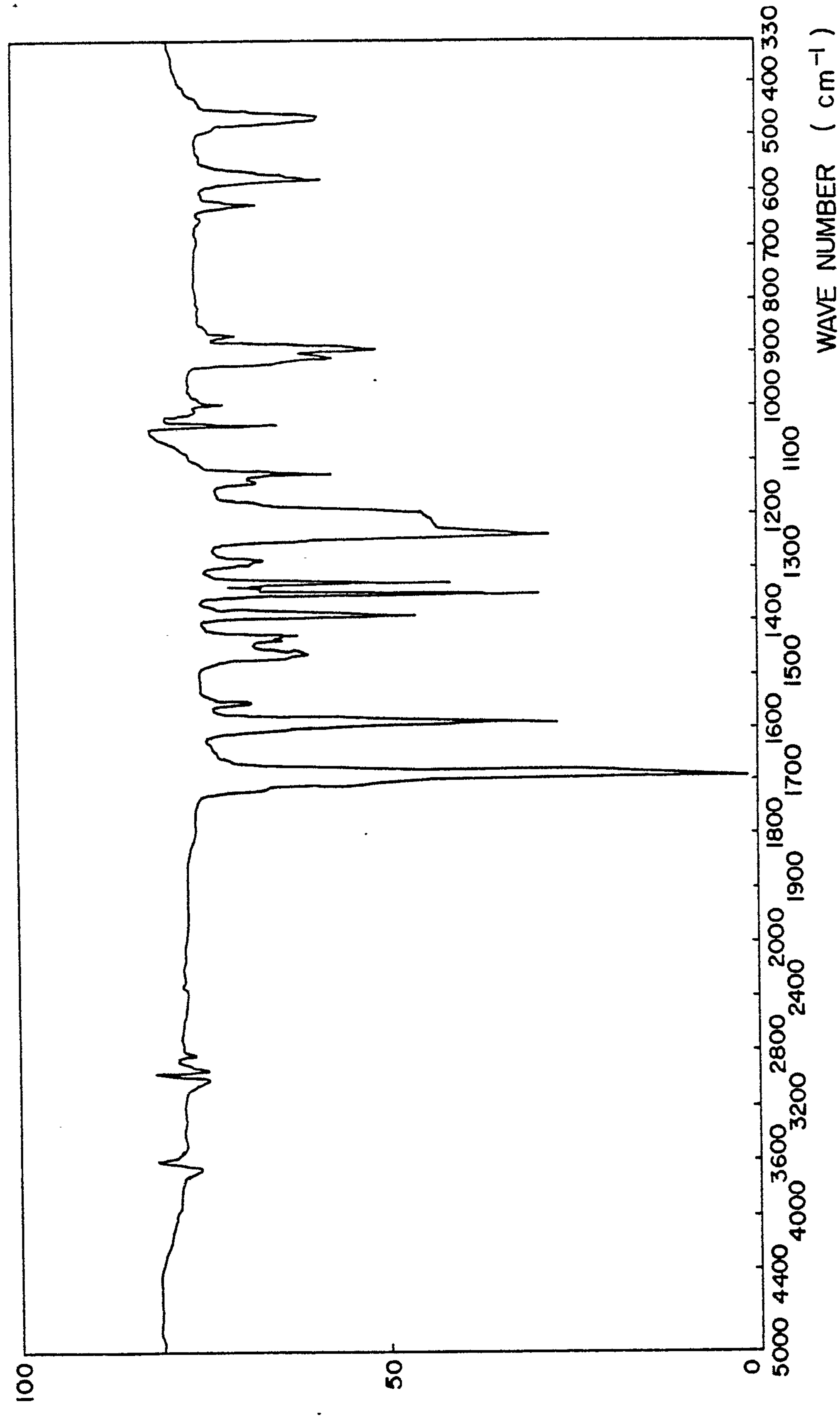
100
50
0
5000 4400 4000 3600 3200 2800 2400 2000 1900 1800 1700 1600 1500 1400 1300 1200 1100 1000 900 800 700 600 500 400 330
WAVE NUMBER ( cm⁻¹ )

FIG. 2

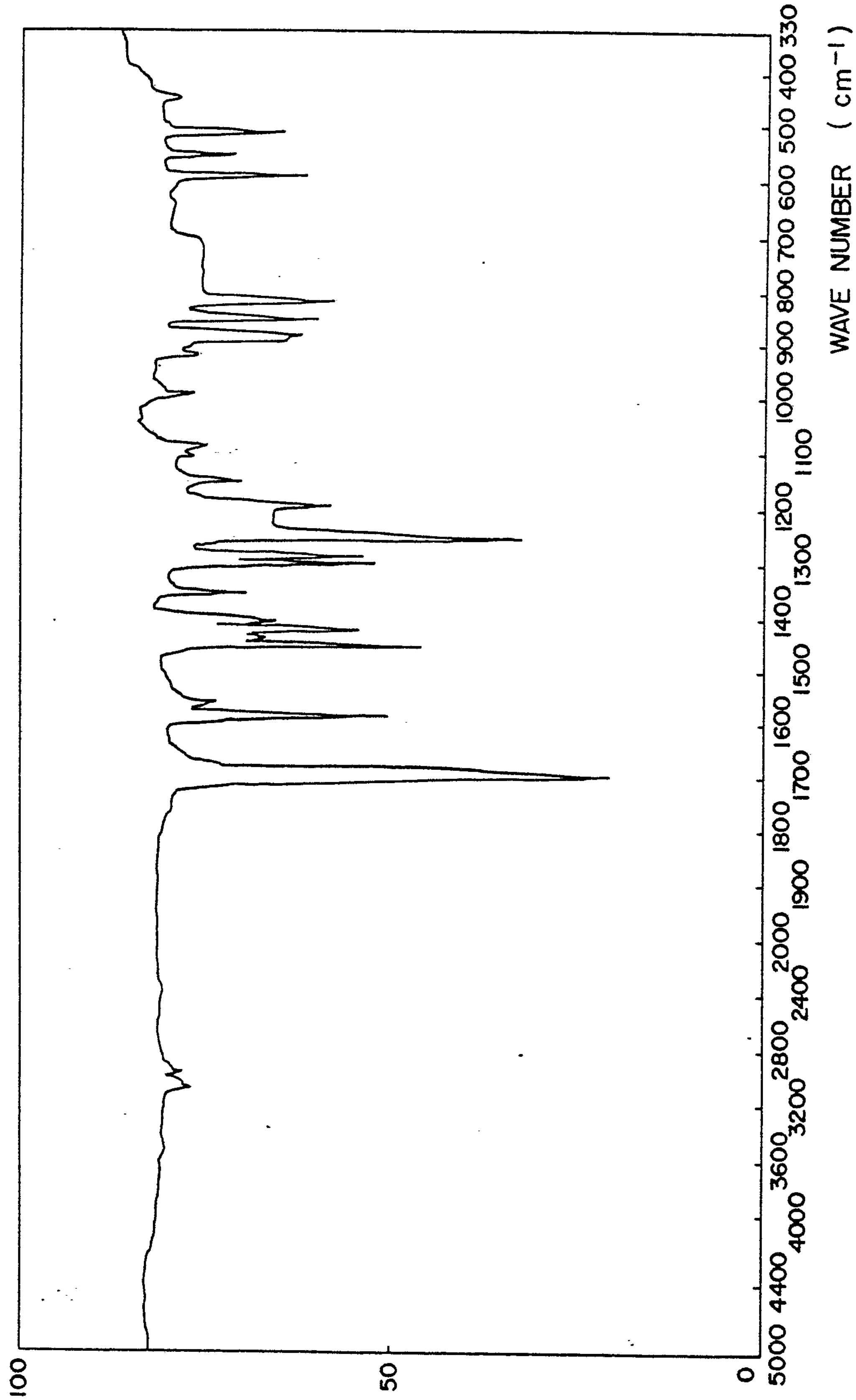
100
50
0
5000 4400 4000 3600 3200 2800 2400 2000 1900 1800 1700 1600 1500 1400 1300 1200 1100 1000 900 800 700 600 500 400 330
WAVE NUMBER ( cm⁻¹ )

FIG. 3

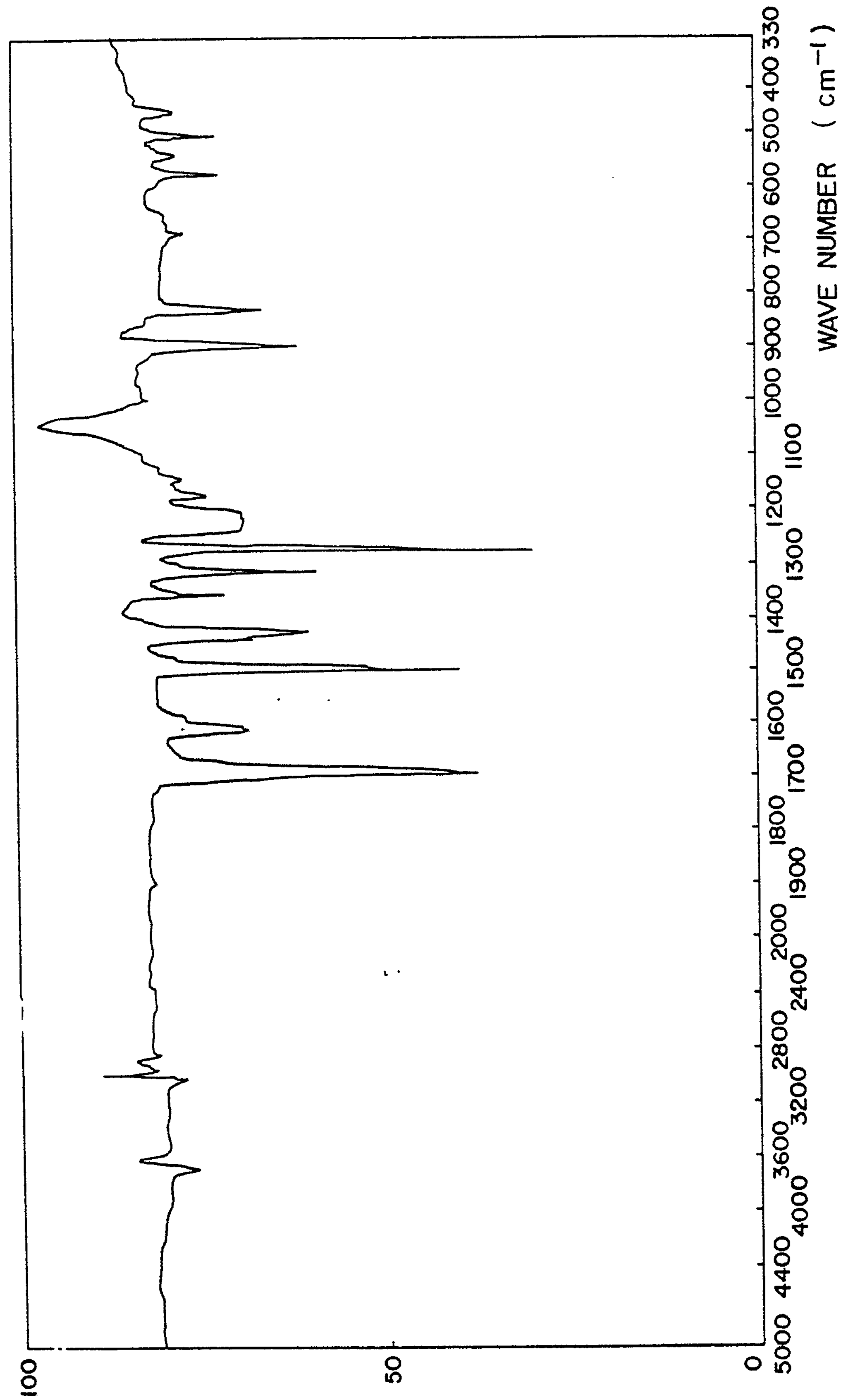
100
50
0
5000 4400 4000 3600 3200 2800 2400 2000 1900 1800 1700 1600 1500 1400 1300 1200 1100 1000 900 800 700 600 500 400 330
WAVE NUMBER (cm⁻¹)

FIG. 4

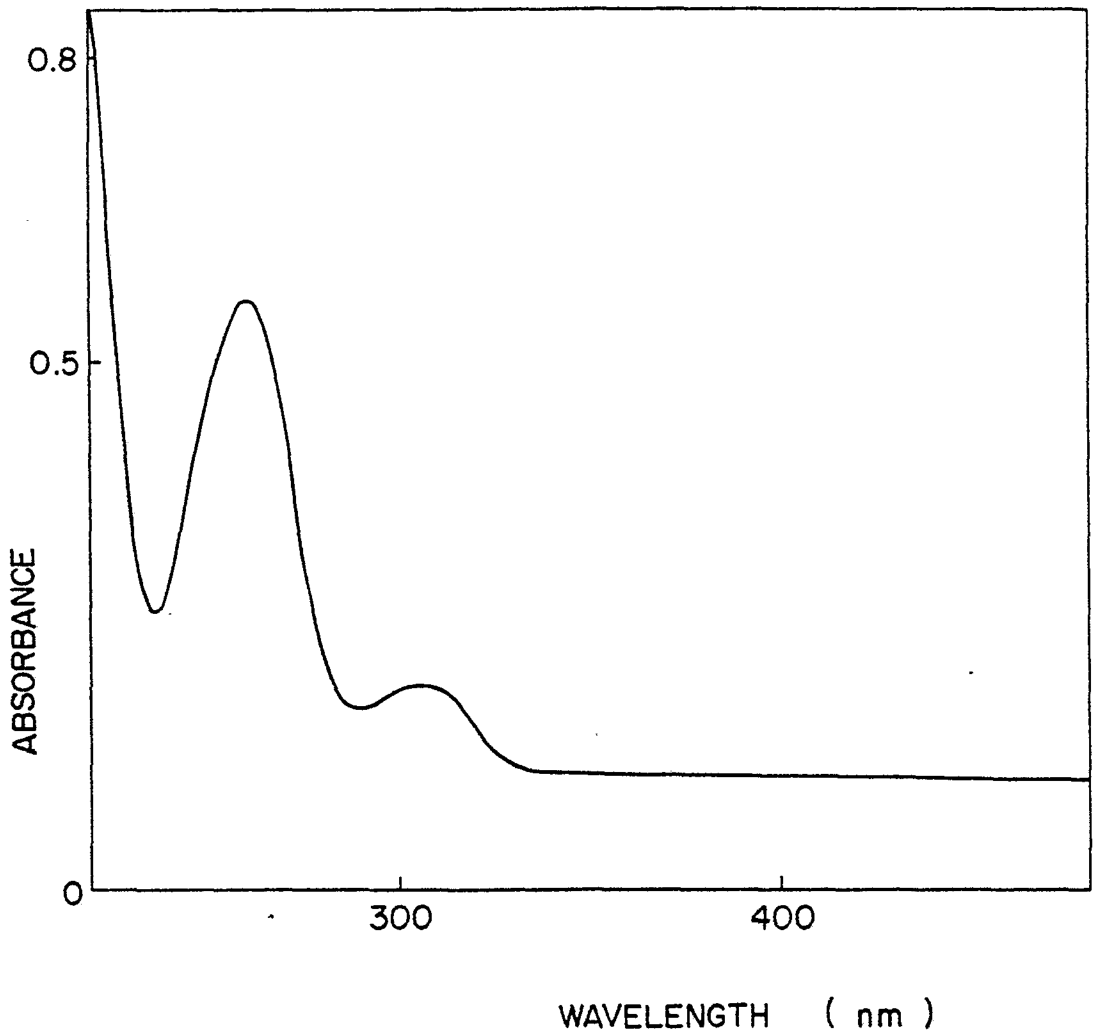

FIG. 5

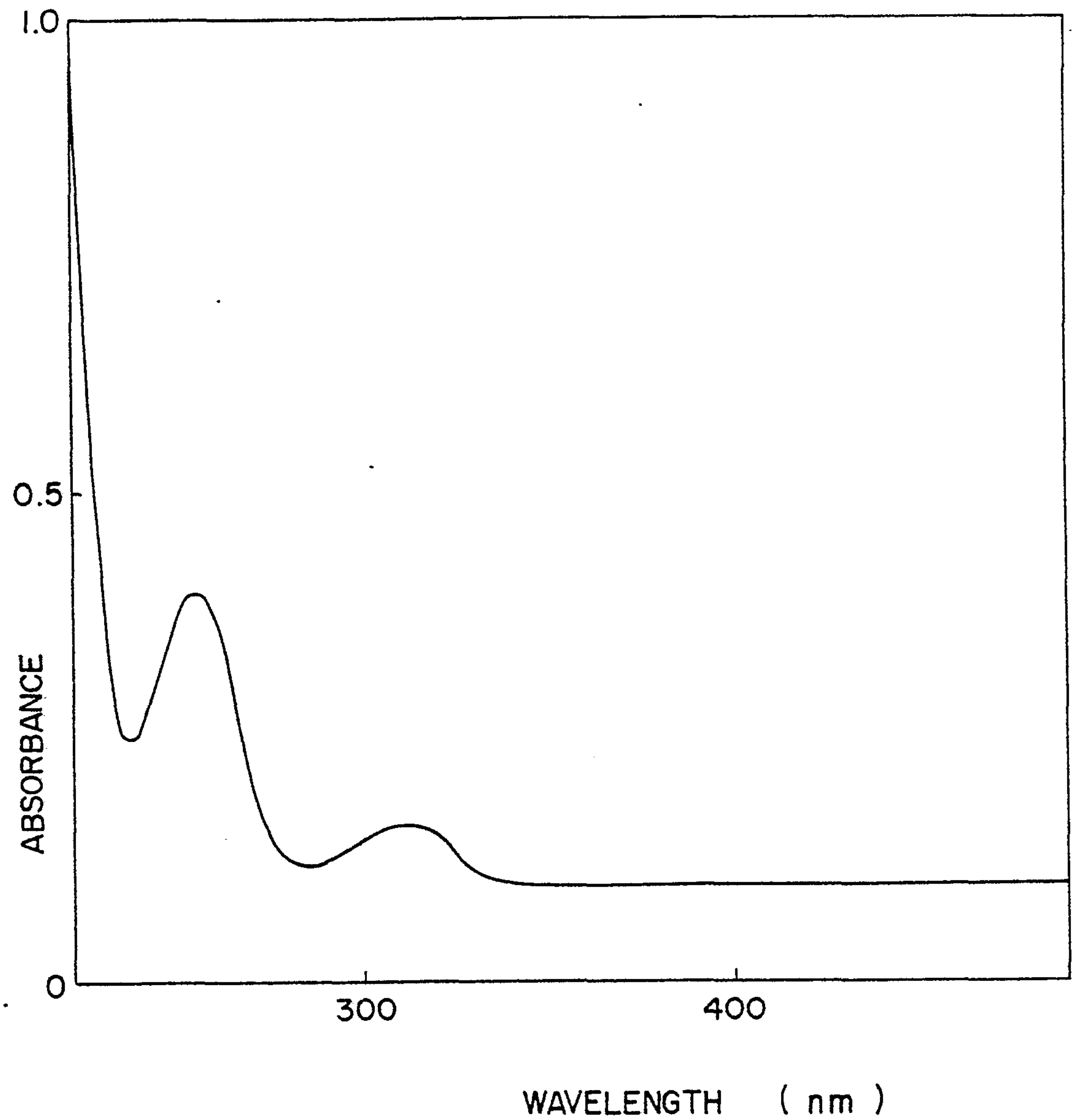

FIG. 6